Europäisches Patentamt

European Patent Office

Office européen des brevets

(19)

(11) Veröffentlichungsnummer: **0 038 985**
**B1**

# (12) EUROPÄISCHE PATENTSCHRIFT

(45) Veröffentlichungstag der Patentschrift:
31.10.84

(51) Int. Cl.³: **B 01 J 13/02,** A 61 K 7/50

(21) Anmeldenummer: **81102793.7**

(22) Anmeldetag: **11.04.81**

(54) **Mikrokapseln mit definierter Öffnungstemperatur, Verfahren zu deren Herstellung sowie deren Verwendung.**

(30) Priorität: **26.04.80 DE 3016170**

(43) Veröffentlichungstag der Anmeldung:
**04.11.81 Patentblatt 81/44**

(45) Bekanntmachung des Hinweises auf die Patenterteilung:
**31.10.84 Patentblatt 84/44**

(84) Benannte Vertragsstaaten:
**DE FR GB IT NL**

(56) Entgegenhaltungen:
**EP - A - 0 009 413**
**AT - A - 352 686**
**DE - A - 2 441 890**
**DE - A - 2 458 879**
**DE - A - 2 515 176**
**DE - A - 2 616 418**
**DE - A - 2 734 577**
**DE - A - 3 032 616**
**FR - A - 2 232 360**
**GB - A - 929 405**

**CHEMICAL ABSTRACTS, Band 91, Nr. 4, 23. Juli 1979,
Seite 64, Nr. 22015q Columbus, Ohio, U.S.A.
SEIFEN- ÖLE - FETTE - WACHSE, Band 98, Nr. 26,
Dezember 1972, Seiten 897-900 R.D. TODD et al.:
"Mikroeingekapselte Parfüme"**

(73) Patentinhaber: **BAYER AG, Konzernverwaltung RP
Patentabteilung, D-5090 Leverkusen 1 Bayerwerk (DE)**

(72) Erfinder: **Schnöring, Hildegard, Dr., Rappenweg 8,
D-5600 Wuppertal 11 (DE)**
Erfinder: **Bömer, Bruno, Dr., Gustav-Freytag-Strasse 2,
D-5090 Leverkusen 1 (DE)**
Erfinder: **Dahm, Manfred, Dr., Am Falkenberg 29,
D-5090 Leverkusen 31 (DE)**

(56) Entgegenhaltungen: (Fortsetzung)
**Die Akte enthält technische Angaben, die nach dem
Eingang der Anmeldung eingereicht wurden und die
nicht in dieser Patentschrift enthalten sind.**

**Beschreibung**

Die vorliegende Erfindung betrifft Mikrokapseln, welche das eingeschlossene Kernmaterial in Gegenwart von Wasser in einem definierten, engen Temperaturintervall abgeben, ein Verfahren zur Herstellung dieser Mikrokapseln sowie deren Verwendung in Waschmitteln und Badezusätzen.

Es ist bereits bekannt geworden, daß sich Mikrokapseln, welche Flüssigkeiten, Feststoffe, Emulsionen oder Suspensionen als Kernmaterialien enthalten und deren Hüllenmaterial aus einer hochmolekularen Substanz besteht, durch Komplexkoazervation herstellen lassen (vgl. DE-B 1 122 495). So ist zum Beispiel möglich, derartige Mikrokapseln herzustellen, indem man die Hüllensubstanz aus wäßriger, gegebenenfalls kolloidaler Lösung auf das in dieser Lösung dispergierte Kernmaterial niederschlägt.

Im einzelnen bereitet man hierbei zunächst eine Dispersion (Emulsion oder Suspension) aus dem gewünschten Kernmaterial und der wäßrigen Lösung der Hüllensubstanz.

Danach wird die Koazervation entweder durch Änderung des pH-Wertes der Lösung oder durch Zugabe von Fällmittel, wie z. B. Wasser oder Methanol, eingeleitet, wobei sich das Koazervat, also die kolloidreiche Phase, d. h. die Hüllensubstanz, aus der kontinuierlichen Phase der Dispersion abscheidet und sich um die diskontinuierliche Phase, d. h. das Kernmaterial, herum ablagert.

Diese Abscheidung des Koazervats um die diskontinuierliche Phase herum ist immer dadurch bedingt, daß die einzelnen Partikel Inhomogenitätsstellen im Kontinuum der kolloidalen Lösung darstellen und daher als Kondensationskeime bei der Bildung der kolloidreichen Phase (des Koazervats) wirken, so daß sich eine geschlossene Koazervathülle um die einzelnen Partikel herum bildet.

Als Hüllenmaterialien für derartige Koazervat-Systeme wurden unter anderem hydrophile, filmbildende Kolloide, wie eine Kombination aus sauer und alkalisch geäscherter Gelatine oder eine Kombination aus sauer geäscherter Gelatine und Gummiarabicum (vgl. DE-B 1 122 495) und ferner Mischungen aus Gelatine und anionischen Polymeren, wie Acrylamid-Maleinsäure-Terpolymerisaten, (vgl. DE-A 2 734 577) beschrieben. Ein entscheidender Nachteil diese bekannten Mikrokapsel-Suspensionen besteht darin, daß sie bei Raumtemperatur nicht lagerstabil sind, sondern zu einer Gallerte zusammenlaufen. Bei Lagertemperaturen unter 10° C bleiben die Kapseln zwar einzeln, aber die Hüllenmaterialien lösen sich bei erhöhten Temperaturen oder bei Änderungen des pH-Wertes der Suspensionen auf und das Kernmaterial wird nicht immer zu dem gewünschten Zeitpunkt und an der gewünschten Stelle freigesetzt.

Weiterhin ist bereits bekannt geworden, daß sich Mikrokapseln, deren Hüllen aus Gelatine bestehen oder Gelatine enthalten, mit Hilfe verschiedener Reagenzien härten lassen.

So eignen sich zum Beispiel Carbonylverbindungen wie Formaldehyd, Glyoxal, Glutardialdehyd oder Pentandion-2,3, um Wandmaterialien auf Gelatine-Basis durch Bildung kovalenter Bindungen irreversibel zu vernetzen und damit zu härten (vgl. Fr-A 2 232 360, EP-A 0 009 413, DE-A 2 616 418 und DE-A 2 515 176). Im allgemeinen wird dabei so verfahren, daß die Härtungsmittel zu der Kapselsuspension gegeben werden und dann der pH-Wert der Suspension auf einen für die Vernetzungsreaktion günstigen Wert, zum Beispiel auf einen pH-Wert zwischen 9 und 10 bei Verwendung von Formaldehyd, eingestellt wird.

Optimal mit Carbonylverbindungen gehärtete Mikrokapselsuspensionen geben ihr Kernmaterial auch bei Temperaturen von 100° C und mehr in Gegenwart von Wasser nicht frei. Auch diese gehärteten Mikrokapseln sind jedoch mit dem Nachteil behaftet, daß sie die Kernmaterialien nicht bei einer definierten hohen Temperatur abgeben. Die Freisetzungstemperatur für das eingeschlossene Kernmaterial steigt vielmehr mit der Lagerungszeit an. Dieser unerwünschte Effekt läßt sich auch durch Abtrennen der wäßrigen Phase mittels Dekantieren oder Abfiltrieren und sorgfältigem Waschen der Kapseln nicht unterdrücken.

Außerdem ist es bekannt, Mikrokapseln, deren Hüllen aus Gelatine bestehen oder Gelatine enthalten, mit Gerbstoffen bzw. mit Aldehyden und Gerbstoffen zu härten (vgl. GB-A 929 405, DE-A 2 441 890, DE-A 3 032 616 und AT-A 352 686). Dabei gilt die Reihenfolge, in der die Härtungsmaterialien eingesetzt werden, als unkritisch. So wird zunächst mit Aldehyd und dann mit Gerbstoff gehärtet (vgl. DE-A 2 441 890, Seite 4, erster Absatz, und DE-A 3 032 616, Seiten 12, 14 und Anspruch 1). Außerdem werden relativ große Mengen an Härtungskomponenten verwendet, und die entstehenden Mikrokapseln geben das eingeschlossene Kernmaterial nicht in einem definierten, engen Temperaturintervall ab.

Es wurden nun Mikrokapseln gefunden, welche das eingeschlossene Kernmaterial in Gegenwart von Wasser in einem definierten, engen Temperaturintervall abgeben. Die erfindungsgemäßen Mikrokapseln sind dadurch charakterisiert, daß das Wandmaterial der Mikrokapseln aus nacheinander mit synthetischen Gerbstoffen sowie mit Glutardialdehyd gehärteter Gelatine im Gemisch mit Carboxymethylcellulose und anionischen Polymeren besteht, wobei die anionischen Polymeren aus

(A) einem Copolymerisat, dessen Struktureinheiten zu 65—90 Mol-%, bezogen auf Polymer, aus statistisch verteilten Resten des Acrylamids und 10—35 Mol-% Resten von Maleinsäure oder Maleinsäureanhydrid bestehen, und das eine Grenzviskositätszahl $[\eta]$ von 0,05 bis 1,0 [dl/g] besitzt, und

(B) einem Copolymerisat, dessen Struktureinheiten aus statistisch verteilten polymerisierten Resten von Acrylamid, Acrylsäure und Maleinsäure bestehen, — wobei die Maleinsäurereste zumindest

2

teilweise in Salzform vorliegen —, das eine Grenzviskositätszahl [$\eta$] von 0,05—1,5 [dl/g] besitzt, und 65 bis 90 Mol-%, bezogen auf Polymer, Reste des Acrylamids und der Acrylsäure zusammen, und 10—35 Mol-% Reste der Maleinsäure enthält,

bestehen, wobei das Gewichtsverhältnis (A) : (B) = 1 : 2 bis 20 : 1 beträgt.

Weiterhin wurde gefunden, daß sich die erfindungsgemäßen Mikrokapseln herstellen lassen, indem man

a) Gelatine im Gemisch mit Carboxymethylcellulose und anionischen Polymeren, die aus
    (A) einem Copolymerisat, dessen Struktureinheiten zu 65—90 Mol-%, bezogen aus Polymer, aus statistisch verteilten Resten des Acrylamids und 10—35 Mol-% Resten von Maleinsäure oder Maleinsäureanhydrid bestehen, und das eine Grenzviskositätszahl [$\eta$] von 0,05 bis 1,0 [dl/g] besitzt, und
    (B) einem Copolymerisat, dessen Struktureinheiten aus statistisch verteilten polymerisierten Resten von Acrylamid, Acrylsäure und Maleinsäure bestehen, — wobei die Maleinsäurereste zumindest teilweise in Salzform vorliegen —, das eine Grenzviskositätszahl [$\eta$] von 0,05—1,5 [dl/g] besitzt, und 65 bis 90 Mol-%, bezogen auf Polymer, Reste des Acrylamids und der Acrylsäure zusammen, und 10—35 Mol-% Reste der Maleinsäure enthält,
    bestehen, wobei das Gewichtsverhältnis (A) : (B) = 1 : 2 bis 20 : 1 beträgt,
    in Gegenwart des Kernmaterials in wäßrigem Medium durch Verdünnung, Abkühlung und/oder durch Änderung des pH-Wertes des Gemisches koazerviert,
b) die Hüllen der dabei entstehenden Mikrokapseln nach in saurem Medium bei pH-Werten zwischen 3,5 und 5,0 vorzunehmender Vorhärtung mit synthetischen Gerbstoffen danach mit Glutardialdehyd im schwach sauren oder basischen Medium bei pH-Werten zwischen 6,0 und 10,0 härtet und
c) schließlich die so erhaltene Mikrokapselsuspension entweder direkt trocknet oder die Mikrokapseln von der flüssigen Phase trennt und dann trocknet.

Außerdem wurde gefunden, daß die erfindungsgemäßen Mikrokapseln je nach der Art des enthaltenen Kernmaterials in Waschmitteln oder Badezusätzen verwendet werden können.

Überraschenderweise besitzen die erfindungsgemäßen Mikrokapseln eine definierte, in einem engen Temperaturintervall liegende Öffnungstemperatur, die sich bei Lagerung nicht oder nur sehr geringfügig ändert. Derartige Eigenschaften konnten im Hinblick auf den bekannten Stand der Technik nicht erwartet werden, denn bei allen chemisch ähnlichen vorbeschriebenen Mikrokapselsuspensionen steigt die Freisetzungstemperatur für das Kernmaterial mit der Lagerungszeit an. Überraschend ist insbesondere, daß die erfindungsgemäßen Mikrokapseln mit den genannten Eigenschaften nur dann entstehen, wenn die Härtung mit synthetischen Gerbstoffen und Glutardialdehyd in der erwähnten Reihenfolge durchgeführt wird und eine abschließende Trocknung erfolgt. Wenn die Härtung zunächst mit Glutardialdehyd und danach mit Gerbstoff vorgenommen wird, oder wenn nach beendeter Herstellung nicht getrocknet wird, dann bilden sich Mikrokapseln, deren Öffnungstemperatur sich verändert (vgl. Beispiele 2 bis 5).

Die erfindungsgemäßen Mikrokapseln zeichnen sich durch mehrere Vorteile aus. So sind sie in einfacher Weise herstellbar und können gelagert werden, ohne daß sich die Temperatur, bei der das Kernmaterial freigesetzt wird, wesentlich ändert. Außerdem lassen sich die in Pulverform vorliegenden erfindungsgemäßen Mikrokapseln bequem verpacken, transportieren sowie mit anderen Pulvern abmischen und jederzeit redispergieren.

Ein besonderer Vorteil besteht darin, daß sich die Freisetzungstemperatur für das Kernmaterial durch die Wahl der Härtungsbedingungen in weiten Grenzen variieren und in der jeweils gewünschten Höhe einstellen läßt.

Als Wandbildungsmaterialien kommen bei der Herstellung der erfindungsgemäßen Mikrokapseln (vorzugsweise sauer oder alkalisch geäscherte) Gelatine im Gemisch mit Carboxymethylcellulose und den oben erwähnten anionischen Polymeren in Frage.

Die als Wandbildungsmaterialien in Betracht kommenden Stoffe und deren Verwendung für diesen Zweck sind bekannt (vgl. DE-B 1 122 495 und DE-A 2 734 577).

Die anionischen Polymeren werden im Falle des erfindungsgemäßen Verfahrens in Form wäßriger Lösungen eingesetzt, die 5 bis 30 Gewichts-%, bezogen auf die Lösung an Polymeren enthalten.

Bei dem erfindungsgemäßen Verfahren werden synthetische Gerbstoffe als Vorhärtungsmittel eingesetzt. Als synthetische Gerbstoffe seien methylenverknüpfte Kondensationsprodukte aus Oxiarylsulfon und aromatischen Sulfonsäuren, wie das unter der Bezeichnung Tanigan® QF bekannte Produkt, und ferner methylenverknüpfte Kondensationsprodukte von aromatischen Sulfonsäuren, wie die unter den Bezeichnungen Tanigan® PR und Tanigan® PT bekannten Produkte, speziell genannt.

Die synthetischen Gerbstoffe führen zu keiner oder nur zu geringer Verfärbung der Kapselsuspensionen.

Nach dem erfindungsgemäßen Verfahren können eine Vielzahl von in Wasser schwer- oder unlöslichen flüssigen oder festen dispergierbaren und gegen Wasser hinreichend beständigen Stoffen mikroverkapselt werden.

Beispielhaft genannt seien organische Lösungsmittel, Paraffinöl, Parfümöle, Silikonentschäumer, Phosphorsäureester, flüssige Kristalle, Farbpigmente, Desinfektionsmittel und Waschmittelzusätze.

Bei der Durchführung des erfindungsgemäßen Verfahrens arbeitet man in wäßrigem Medium.

Die Koazervierung wird bei dem erfindungsgemäßen Verfahren entweder durch Verdünnung des Reaktionsgemisches, Abkühlung des Reaktionsgemisches oder durch Änderung des pH-Wertes des Reaktionsgemisches oder auch durch eine Kombination dieser Maßnahmen durchgeführt. Als Verdünnungsmittel kommen hierbei vorzugsweise Wasser und/oder mit Wasser mischbare Alkohole, wie Methanol oder Ethanol, in Frage. Eine Änderung des pH-Wertes kann, je nach dem verwendeten Kolloid, durch Zusatz von Säure oder von Base erfolgen.

Die Koazervierung kann bei dem erfindungsgemäßen Verfahren innerhalb eines größeren Temperaturbereiches erfolgen. Im allgemeinen arbeitet man bei Temperaturen zwischen 0°C und 80°C, vorzugsweise zwischen 5°C und 60°C.

Die Vorhärtung der Mikrokapseln erfolgt bei dem erfindungsgemäßen Verfahren im sauren Medium bei pH-Werten zwischen 3,5 und 5,0.

Bei der Vorhärtung kann die Temperatur innerhalb eines bestimmten Bereiches variiert werden. Im allgemeinen arbeitet man bei Temperaturen zwischen 5°C und 70°C, vorzugsweise zwischen 20°C und 50°C.

Die Härtung mit Glutardialdehyd wird im basischen oder schwach sauren Medium vorgenommen. Weist die vorgehärtete Kapselsuspension einen pH-Wert im sauren Bereich auf, so wird der gewünschte pH-Wert durch Zugabe von Basen eingestellt. Als Basen kommen hierbei vorzugsweise wäßrige Natronlauge, wäßrige Kalilauge und wäßrige Sodalösung in Frage.

Bei dieser Härtung arbeitet man bei pH-Werten zwischen 6,0 und 10,0.

Die Temperatur kann bei der Härtung innerhalb eines bestimmten Bereiches variiert werden. Im allgemeinen arbeitet man bei Temperaturen zwischen 0°C und 70°C, vorzugsweise zwischen 20°C und 60°C.

Die Trocknung der erfindungsgemäß herstellbaren Mikrokapselsuspensionen erfolgt entweder direkt oder nach Abtrennung der Mikrokapseln von der flüssigen Phase. Eine direkte Trocknung kann zum Beispiel im Sprühtrockner oder im Fließbett vorgenommen werden. Die anfängliche Produkttemperatur (Trockengut-Temperatur) sollte immer um mindestens 20°C unter derjenigen Temperatur liegen, bei der das Kernmaterial aus den erfindungsgemäßen Mikrokapseln freigesetzt wird. Mit zunehmender Trocknungsdauer kann die Trockengut-Temperatur erhöht werden. Im allgemeinen liegt die End-Trocknungstemperatur der Mikrokapseln bei 70°C.

Es ist auch möglich, die Mikrokapseln zunächst zum Beispiel durch Dekantieren weitgehend von der flüssigen Phase abzutrennen und anschließend, — gegebenenfalls nach Abmischung mit einem Fließhilfsmittel wie hochdisperser Kieselsäure, Bariumstearat oder Kreide —, zu trocknen.

Bei der Durchführung des erfindungsgemäßen Verfahrens kann das Vorhärtungsmittel während oder nach der Koazervierung zugegeben werden. Besonders vorteilhaft ist es, das Vorhärtungsmittel mit dem Verdünnungsmittel, also bevorzugt mit dem zur Verdünnung dienenden Wasser, zuzugeben, da man bei dieser Verfahrensweise besonders wenig Leerkoazervat, d. h. Wandmaterial ohne eingeschlossenes Kernmaterial, erhält.

Der als eigentliches Härtungsmittel fungierende Glutardialdehyd wird nach vorher erfolgter Vorhärtung in einer sich daran anschließenden Phase zugegeben, in der die Kapselsuspension auf niedrige Temperaturen, bevorzugt zwischen 5°C und 10°C, abgekühlt wird.

Die Härtungswirkung des Glutardialdehydes hängt vom pH-Wert der Kapselsuspension ab. Der günstigste pH-Bereich liegt zwischen 6,0 und 10,0.

Die erfindungsgemäßen Mikrokapseln geben ihr Kernmaterial in Gegenwart von Wasser oder wäßrigen Lösungen in einem definierten, engen Temperaturintervall (= Öffnungstemperatur, Freisetzungstemperatur) ab. Dabei kann die Öffnungstemperatur der Mikrokapseln innerhalb eines relativ großen Bereiches gezielt eingestellt werden. Im allgemeinen liegt die Öffnungstemperatur in einem bestimmten engen Temperaturintervall bei Temperaturen zwischen 30°C und 150°C, vorzugsweise zwischen 35°C und 120°C. Die jeweils gewünschte Öffnungstemperatur wird dadurch erzielt, daß man die Mikrokapselsuspensionen nach der Koazervierung so weit härtet bis die gewünschte Freisetzungstemperatur erreicht ist und die Mikrokapselsuspensionen dann trocknet. Die Öffnungstemperatur der getrockneten Kapseln liegt im allgemeinen um 2 bis 5°C höher als die der Kapselsuspension (Kapselslurry) vor der Trocknung.

Läßt man auf nach der Koazervierung und Vorhärtung anfallende Mikrokapselsuspensionen eine definierte Menge Glutardialdehyd bei einem bestimmten pH-Wert und einer bestimmten Temperatur einwirken, so nimmt die Freisetzungstemperatur für das Kernmaterial mit zunehmender Einwirkungsdauer des Glutardialdehydes zu. Bei gleicher Einwirkungsdauer und Härtungsmittelkonzentration nimmt die Freisetzungstemperatur in bestimmten Bereichen mit steigendem pH-Wert und steigender Reaktionstemperatur zu.

Die Temperatur, bei der die erfindungsgemäßen Mikrokapseln das Kernmaterial abgeben, läßt sich in einfacher Weise bestimmen. Man verfährt dabei so, daß man in einem Vorversuch das Kernmaterial mit einem öllöslichen aber in Wasser unlöslichen Farbstoff anfärbt und dann nach dem erfindungsgemäßen Verfahren einkapselt. Nach einer kurzen Zeit werden 20 ml der gehärteten Mikrokapselslurry in 80 ml entmineralisiertes Wasser gegeben, und die Slurry wird 5 Minuten bei einer deutlich unter der zu erwartenden Öffnungstemperatur liegenden Temperatur gerührt. Dann werden 1,5 ml dieser Kapselsuspension in 35—45°C warmes, mit Wasser nicht mischbares organisches Lösungsmittel gegeben, umgeschüttelt, und nach dem Absinken der Kapseln wird die Farbe der organischen Flüssigkeit beurteilt. Die Slurry wird dann bei einer 1—3°C höheren Temperatur wieder 5 Minuten gerührt und erneut auf Dichtigkeit der Kapseln geprüft. Die Öffnungstemperatur ist daran erkennbar, daß sich das organische Lösungsmittel durch Aufnahme des aus den Kapseln tretenden Farbstoffes verfärbt. Als öllöslicher und in Wasser unlöslicher Farbstoff kann zum Beispiel 1-Oxy-4-p-toluylamino-anthrachinon (=Macro-Lexviolett B®) verwendet werden. Ein geeignetes organisches Lösungsmittel, durch welches aus den intakten Kapseln auch nach längerem Stehen kein Farbstoff extrahiert wird, ist Ethylacetat.

Bei der Bestimmung der Öffnungstemperatur nach der beschriebenen Methode wird die maximale Verfärbung des organischen Lösungsmittels im allgemeinen in einem Temperaturintervall von 1 bis 3°C deutlich sichtbar. Je höher die Öffnungstemperatur der Mikrokapseln liegt, desto breiter ist in der Regel der Temperaturbereich, in dem eine beginnende Verfärbung der organischen Lösungsmittelphase zu beobachten ist.

Liegt die ermittelte Öffnungstemperatur unter der gewünschten, so wird die Härtung der Kapseln so lange fortgesetzt, bis die Öffnungstemperatur der Kapseln in der Suspension um 2—4°C unter der gewünschten End-Öffnungstemperatur der Kapseln liegt. Danach wird die Weiterhärtung der Kapseln durch Trocknen beendet.

Bei der Durchführung des erfindungsgemäßen Verfahrens geht man bevorzugt so vor, daß man die Hüllen- und Kernmaterialien in wäßriger Lösung in dem jeweils gewünschten Mengenverhältnis bei Temperaturen zwischen 20°C und 60°C unter Rühren bis zum Erreichen einer mittleren Partikelgröße von z. B. 10 bis 15 µm emulgiert, dann zur Einleitung der Koazervation eine erwärmte, synthetischen Gerbstoff enthaltende wäßrige Lösung einrührt, danach das Gemisch auf Temperaturen zwischen 5°C und 10°C abkühlt und nach 1 bis 4 Stunden mit einer wäßrigen Glutardialdehyd-Lösung versetzt, anschließend durch Zugabe einer wäßrigen Base den pH-Wert des Gemisches in den gewünschten Bereich bringt, dann nachrührt und die entstehende Kapselsuspension mit Hilfe eines Sprühtrockners oder im Fließbett trocknet.

Die erfindungsgemäßen Mikrokapseln lassen sich, wie bereits erwähnt, für eine Vielzahl von Zwecken einsetzen. Beispielsweise genannt sei die Verwendung in Waschmitteln und Badezusätzen. Zu einem Einsatz in Waschmitteln kommen solche erfindungsgemäßen Mikrokapseln in Frage, die in einem bestimmten Temperaturintervall zwischen 40°C und 95°C das Kernmaterial freigeben. Mikrokapseln, die in Badezusätzen Verwendung finden, besitzen Öffnungstemperaturen zwischen 35 und 40°C.

Die Herstellung und die Eigenschaften der erfindungsgemäßen Mikrokapseln gehen aus den nachfolgenden Beispielen hervor.

## Beispiel 1

### I) Herstellung der anionischen Polymerlösung

a)   Polymerisation: (Copolymerisat (A))
     225 g Acrylamid, 75 g Maleinsäureanhydrid und 3 g Azoisobuttersäuredinitril werden in 2,7 l Essigsäureethylester gelöst. Die Lösung wird durch mehrmaliges Evakuieren der Apparatur und Füllen mit Stickstoff von Sauerstoff befreit und unter Sauerstoffausschluß 20 Stunden bei 60°C gerührt. Das ausgefallene Polymerisat wird abfiltriert, gründlich mit Essigsäureethylester gewaschen und im Vakuum bei 60°C getrocknet. Man erhält 273 g eines feinpulvrigen Polymeren mit einer in 0,9%iger wäßriger NaCl-Lösung bestimmten Viskositätszahl (intrinsic viscosity) $[\eta] = 0,14$ [dl/g].

b)   Hydrolyse: (Copolymerisat (B))
     100 g des trockenen Polymeren gemäß I a) werden in 900 ml entmineralisiertem Wasser gelöst, und die Lösung wird bis zum pH-Wert 4,6 bei Normaldruck unter Rückfluß erhitzt (ca. 12—18 Stunden).

c)   Polymerlösung
     150 g trockenes Polymerisat gemäß Ia werden in 1350 ml entmineralisiertem Wasser gelöst. Die Lösung zeigt einen pH-Wert von ~2,2. 300 ml dieser Lösung werden mit 75 ml der hydrolysierten Lösung gemäß Ib gemischt. Die Mischung besitzt einen pH-Wert von 3,4.

### II) Herstellung der Mikrokapseln

300 ml einer 10gew.-%igen wäßrigen Lösung von saurer geäscherter Schweinehautgelatine und 300 ml einer 10%igen wäßrigen, gemäß Ic hergestellten Polymerlösung werden bei 50°C zusammengegeben. In die entstehende klare Lösung werden 240 g Wärmeübertragungsöl Marlotherm® S, das mit 0,5 g des öllöslichen Farbstoffes Macro-Lexviolett B® (= 1-Oxy-4-p-toluylamino-anthrachinon) angefärbt ist, bis zu einer mittleren Tropfengröße von 15 μm einemulgiert. Dann wird eine auf 50°C erwärmte Lösung von 1,3 g Carboxymethylcellulose und 1,2 g Tangian QF® in 600 ml Wasser eingerührt. Anschließend wird die Mischung unter Rühren auf 8°C abgekühlt. Nach 3 Stunden bei 8 bis 10°C werden 195 g einer 0,1gew.-%igen wäßrigen Lösung von Glutardialdehyd zugefügt. Es wird 2 bis 3 Minuten nachgerührt, der pH-Wert des Gemisches wird mit 10%iger wäßriger Natronlauge auf 6,5 gestellt, und die Slurry wird 16 Stunden bei Raumtemperatur nachgerührt.

Die Öffnungstemperatur der nicht getrockneten Kapseln beträgt 48 bis 50°C.

Durch Trocknung der Kapseln im Fließbett oder im Sprühtrockner erhält man ein freifließendes Pulver, welches sich auch nach einhunderttägiger Lagerung bei 50°C leicht in Wasser dispergieren läßt und dann eine Öffnungstemperatur von 52—54°C besitzt.

Die Trocknung wird im vorliegenden Fall in einem Zerstäubungstrockner mit Zweistoffdüse unter Verwendung von Stickstoff als Treibgas bei einer Heißgastemperatur von 130°C und einer Ablufttemperatur von 65°C durchgeführt. Dabei ist es vorteilhaft, die Kapselslurry auf etwa 4°C vorzukühlen.

## III) Bestimmung der Öffnungstemperatur

a) Zur Bestimmung der Öffnungstemperatur der gemäß II hergestellten, in Suspension vorliegenden und zuvor nicht getrockneten Mikrokapseln werden 20 ml der gehärteten Kapselslurry in 80 ml entmineralisiertes Wasser gegeben. Die Slurry wird 5 Minuten bei 30°C gerührt. Dann werden 1,5 ml der Kapselsuspension in 20 ml auf 30°C erwärmtes Ethylacetat gegossen, und es wird umgeschüttelt.

Nach dem Absetzen der Kapseln wird die Farbe der Ethylacetat-Lösung beurteilt. Aus den noch intakten Kapseln wird auch bei längerem Stehen in Ethylacetat kein Farbstoff extrahiert. Danach wird die Temperatur der Suspension um jeweils 1 bis 2°C gesteigert und unter Rühren jeweils 5 Minuten konstant gehalten. Es wird jeweils erneut auf Dichtheit der Kapseln geprüft, indem 1,5 ml der Kapseln in Ethylacetat von 30—40°C gegeben werden und nach Umschütteln und Absitzen der Kapseln die Färbung der Ethylacetat-Phase beurteilt wird. In dem Temperaturbereich von 48 bis 50°C wird die maximale Blaufärbung beobachtet.

b) Zur Prüfung der Öffnungstemperatur der gemäß II hergestellten getrockneten Mikrokapseln verfährt man in der Weise, daß man 4 g der getrockneten Kapseln in 100 ml entmineralisiertem Wasser suspendiert und dann so vorgeht, wie es unter III-a beschrieben wurde. Die Öffnungstemperatur der Kapseln liegt zwischen 52 und 54°C.

## Beispiele 2—5

Einfluß des pH-Wertes bei der Härtung auf die Öffnungstemperatur der Kapseln.

Man verfährt wie im Beispiel 1 unter II beschrieben, setzt aber den pH-Wert der Suspension bei der Härtung mit Glutardialdehyd durch Zugabe von 10%iger wäßriger Natronlauge auf die in der folgenden Tabelle 1 angegebenen pH-Werte.

Tabelle 1

| Beispiel Nr. | pH-Wert | Öffnungstemperatur in °C | | | |
| | | A | B | C | D |
|---|---|---|---|---|---|
| 2 | 6,56 | 52 | 52 | 53 | >100 |
| 3 | 6,61 | 54 | 56 | 56 | >100 |
| 4 | 6,72 | 55 | 57 | 57 | >100 |
| 5 | 6,87 | 58 | 61 | 61 | >100 |

A = Slurry nach der Herstellung
B = Pulver nach der Herstellung
C = Pulver nach 6 Monaten Lagerung bei Raumtemperatur
D = Slurry nach 6 Monaten Lagerung bei Raumtemperatur
(Kapseln nach Herstellung nicht getrocknet)

### Beispiele 6—24

Einfluß der Konzentration von Glutardialdehyd und der Art des im sauren pH-Bereich wirksamen Gerbstoffs auf die Öffnungstemperatur der Mikrokapseln.

Jeweils 300 ml einer 10gew.-%igen wäßrigen Lösung von saurer geäscherter Schweinehautgelatine im 300 ml einer 10%igen wäßrigen, gemäß Beispiel 1 (Ic) hergestellten Polymerlösung werden bei 50°C zusammengegeben. In die jeweils entstehende klare Lösung werden jeweils 240 g Wärmeübertragungsöl Marlotherm® S, das mit 0,5 g des öllöslichen Farbstoffes Macro-Lexviolett B® (=1-Oxy-4-p-toluylamino-anthrachinon) angefärbt ist, bis zu einer mittleren Tropfengröße von 10 μm einemulgiert. Dann wird eine auf 50°C erwärmte Lösung von 1,3 g Carboxymethylcellulose und 180 mg, entsprechend 0,3% bezogen auf Gelatine und Polymerem, des jeweils verwendeten Gerbstoffes eingerührt.

Anschließend wird die jeweilige Mischung unter Rühren auf 5 bis 10°C abgekühlt. Nach 3 Stunden bei 15 bis 20°C werden jeweils 200 ml einer wäßrigen Glutardialdehyd-Lösung einzugefügt, welche die jeweils in der nachfolgenden Tabelle 2 angegebene Menge an Glutardialdehyd enthält (150 mg=0,25%, 180 mg=0,3%, 210 mg=0,35%, 240 mg=0,4% oder 270 mg=0,45% Glutardialdehyd, bezogen auf die Menge an eingesetzter Gelatine und Polymerem). Es wird 2 Minuten nachgerührt, der pH-Wert des Gemisches wird mit 15gew.-%iger wäßriger Natronlauge auf 6,5 gestellt. Die Slurry wird 5 Minuten nachgerührt und dann 16 Stunden bei 22°C stehengelassen. Anschließend wird die Öffnungstemperatur der Kapseln nach der im Beispiel 1 angegebenen Methode bestimmt. Die Versuchsergebnisse sind in der Tabelle 2 zusammengestellt. Nach der Sprühtrocknung liegen die Öffnungstemperaturen der Kapseln jeweils um 2—5°C höher als in der Slurry kurz vor dem Trocknen.

### Tabelle 2

Einfluß der Konzentration von Glutardialdehyd und der Art des im sauren pH-Bereich wirksamen Gerbstoffs auf die Öffnungstemperatur der Slurry nach 16stündiger Härtung bei 22°C.

| | Beispiel Nr. | | | | | | | | | | | |
|---|---|---|---|---|---|---|---|---|---|---|---|---|
| | 6 | 7 | 8 | 9 | 10 | 11 | 12 | 13 | 14 | 15 | 16 | 17 |
| Tanigan(R)QF*), % | — | — | — | — | 0,3 | 0,3 | 0,3 | 0,3 | 0,3 | — | — | — |
| Tanigan(R)PR*), % | — | — | — | — | — | — | — | — | — | 0,3 | 0,3 | 0,3 |
| Tanigan(R)PT*), % | — | — | — | — | — | — | — | — | — | — | — | — |
| Glutardialdehyd, % | 0,3 | 0,35 | 0,4 | 0,45 | 0,25 | 0,3 | 0,35 | 0,4 | 0,45 | 0,25 | 0,3 | 0,35 |
| pH bei der Härtung | 6,6 | 6,4 | 6,45 | 6,4 | 6,6 | 6,4 | 6,4 | 6,4 | 6,45 | 6,45 | 6,5 | 6,55 |
| Öffnungstemperatur der Slurry (°C) | 56 | 79 | 90 | >90 | 46 | 50 | 66 | 80 | 90 | 41 | 44 | 54 |

*) Gewichts.-% bezogen auf die Summe von Gelatine und Copolymer

### Tabelle 2 (Fortsetzung)

| | Beispiel Nr. | | | | | | |
|---|---|---|---|---|---|---|---|
| | 18 | 19 | 20 | 21 | 22 | 23 | 24 |
| Tanigan(R)QF*), % | — | — | — | — | — | — | — |
| Tanigan(R)PR*), % | 0,3 | 0,3 | — | — | — | — | — |
| Tanigan(R)PT*), % | — | — | 0,3 | 0,3 | 0,3 | 0,3 | 0,3 |
| Glutardialdehyd, % | 0,4 | 0,45 | 0,25 | 0,3 | 0,35 | 0,4 | 0,45 |
| pH bei der Härtung, | 6,5 | 6,5 | 6,5 | 6,45 | 6,5 | 6,45 | 6,45 |
| Öffnungstemperatur der Slurry (°C) | 74 | 90 | 41 | 43 | 51 | 68 | 88 |

*) Gewichts.-% bezogen auf die Summe von Gelatine und Copolymer

### Beispiele 25—39

Einfluß der Konzentration von Gerbstoff [Tanigan®QF] und Glutardialdehyd auf die Öffnungstemperatur der Kapseln.

Jeweils 300 ml einer 10gew.-%igen wäßrigen Lösung von saurer geäscherter Schweinehautgelatine und 300 ml einer 10%igen wäßrigen, gemäß Beispiel 1 (Ic) hergestellten Polymerlösung werden bei 50°C zusammengegeben. In die jeweils entstehende klare Lösung werden jeweils 240 g Wärmeübertragungsöl Marlotherm®S, das mit 0,5 g des öllöslichen Farbstoffes Macro—Lexviolett B® ( = 1-Oxy-4-p-toluylamino-anthrachinon) angefärbt ist, bis zu einer mittleren Tropfengröße von 10 μm einemulgiert. Dann wird eine auf 50°C erwärmte Lösung von 1,3 g Carboxymethylcellulose und die in der nachfolgenden Tabelle 3 jeweils angegebene Menge an Gerbstoff eingerührt (0 g, 60 mg ≙ 0,1%, 120 mg ≙ 0,3% Tanigan® QF, bezogen auf die Summe an eingesetzter Menge an Gelatine und Polymerem).

Nach 3 Stunden bei 15—20°C werden jeweils 200 ml einer wäßrigen Glutardialdehyd-Lösung hinzugefügt, welche die jeweils in der nachfolgenden Tabelle 3 angegebene Menge an Glutardialdehyd enthält (150 mg ≙ 0,25%, 180 mg ≙ 0,3%, 210 mg ≙ 0,35%, 240 mg ≙ 0,4% oder 270 mg ≙ 0,45% Glutardialdehyd, bezogen auf die Menge an eingesetzter Gelatine und Polymerem).

Es wird 2 Minuten nachgerührt, der pH-Wert des Gemisches wird mit 10gew.-%iger wäßriger Natronlauge auf 6,5 gestellt. Die Slurry wird 5 Minuten nachgerührt und dann 16 Stunden bei 22°C stehengelassen. Anschließend wird die Öffnungstemperatur der Kapseln nach der im Beispiel 1 angegebenen Methode bestimmt. Die Versuchsergebnisse sind in der Tabelle 3 zusammengestellt.

### Tabelle 3

Einfluß der Konzentration von Gerbstoff Tanigan[(R)] QF und Glutardialdehyd auf die Öffnungstemperatur der Kapselslurry.

| | Beispiel Nr. | | | | | | | | | | | | | | |
|---|---|---|---|---|---|---|---|---|---|---|---|---|---|---|---|
| | 25 | 26 | 27 | 28 | 29 | 30 | 31 | 32 | 33 | 34 | 35 | 36 | 37 | 38 | 39 |
| Tanigan[(R)]QF*), % | 0 | 0 | 0 | 0 | 0 | 0,1 | 0,1 | 0,1 | 0,1 | 0,1 | 0,2 | 0,2 | 0,2 | 0,2 | 0,2 |
| Glutardialdehyd, % | 0,25 | 0,3 | 0,35 | 0,4 | 0,45 | 0,25 | 0,3 | 0,35 | 0,4 | 0,45 | 0,25 | 0,3 | 0,35 | 0,4 | 0,45 |
| pH bei der Härtung | 6,5 | 6,45 | 6,5 | 6,5 | 6,4 | 6,45 | 6,4 | 6,4 | 6,4 | 6,5 | 6,6 | 6,4 | 6,45 | 6,4 | 6,5 |
| Öffnungstemperatur der Slurry (°C) | 38 | 40 | 49 | 59 | 78 | 40 | 44 | 51 | 70 | 90 | 42 | 47 | 58 | 75 | 90 |

*) Gew.-% bezogen auf die Summe von Gelatine und Copolymer

### Beispiele 40—53

Einfluß des pH-Wertes während der Härtung auf die Öffnungstemperatur der Mikrokapseln.

Jeweils 300 ml einer 10gew.-%igen wäßrigen Lösung von sauer geäscherter Schweinehautgelatine und 300 ml einer 10%igen wäßrigen, gemäß Beispiel 1 (Ic) hergestellten Polymerlösung werden bei 50°C zusammengegeben. In die jeweils entstehende klare Lösung werden jeweils 240 g Wärmeübertragungsöl Marlotherm® S, das mit 0,5 g des öllöslichen Farbstoffes Macro-Lexviolett B® ( = 1-Oxy-4-p-toluylamino-anthrachinon) angefärbt ist, bis zu einer mittleren Tropfengröße von 10 μm einemulgiert.

Dann wird eine auf 50°C erwärmte Lösung von 1,3 g Carboxymethylcellulose und 120 mg ( = 0,2%) Tanigan QF® in 600 ml Wasser eingerührt. Anschließend wird die Mischung unter Rühren auf 5—10°C abgekühlt. Nach 3 Stunden bei 15 bis 20°C werden 200 g einer wäßrigen Lösung von Glutardialdehyd zugefügt, welche 120 mg ≙ 0,2 Gew.-% oder 150 mg ≙ 0,25 Gew.-% an Glutardialdehyd enthält, bezogen auf die Summe der eingesetzten Menge an Gelatine und Polymerem.

Es wird 2 Minuten nachgerührt, der pH-Wert des Gemisches wird mit 10gew.-%iger Natronlauge auf die in der nachfolgenden Tabelle 4 angegebenen Werte eingestellt. Die Slurry wird 5 Minuten nachgerührt und dann 16 Stunden bei 22°C stehengelassen. Anschließend wird die Öffnungstemperatur der Kapseln nach der im Beispiel 1 angegebenen Methode bestimmt. Die Versuchsergebnisse sind in der Tabelle 4 zusammengestellt.

Tabelle 4

Einfluß des pH-Wertes bei der Härtung mit Glutardialdehyd auf die Öffnungstemperatur der Kapselslurry.

| | Beispiel Nr. | | | | | | | | | | | | | |
|---|---|---|---|---|---|---|---|---|---|---|---|---|---|---|
| | 40 | 41 | 42 | 43 | 44 | 45 | 46 | 47 | 48 | 49 | 50 | 51 | 52 | 53 |
| Tanigan(R)QF*), % | 0,2 | 0,2 | 0,2 | 0,2 | 0,2 | 0,2 | 0,2 | 0,2 | 0,2 | 0,2 | 0,2 | 0,2 | 0,2 | 0,2 |
| Glutardialdehyd, % | 0,2 | 0,2 | 0,2 | 0,2 | 0,2 | 0,2 | 0,2 | 0,2 | 0,25 | 0,25 | 0,25 | 0,25 | 0,25 | 0,25 |
| pH bei der Härtung | 6,7 | 7,0 | 7,15 | 7,35 | 7,65 | 7,9 | 8,25 | 8,5 | 6,6 | 6,85 | 7,0 | 7,35 | 7,55 | 7,9 |
| Öffnungs-temperatur, (°C) | 40 | 42 | 43 | 46 | 55 | 69 | >90 | >90 | 43 | 49 | 58 | 72 | 83 | 90 |

*) Gew.-% bezogen auf die Summe von Gelatine und Copolymer

**Patentansprüche**

1. Mikrokapseln, welche das eingeschlossene Kernmaterial in Gegenwart von Wasser in einem definierten, engen Temperaturintervall abgeben, dadurch gekennzeichnet, daß das Wandmaterial der Mikrokapseln aus nacheinander mit synthetischen Gerbstoffen sowie mit Glutardialdehyd gehärteter Gelatine im Gemisch mit Carboxymethylcellulose und anionischen Polymeren besteht, wobei die anionischen Polymeren aus

(A) einem Copolymerisat, dessen Struktureinheiten zu 65—90 Mol-%, bezogen auf Polymer, aus statistisch verteilten Resten des Acrylamids und 10—35 Mol-% Resten von Maleinsäure oder Maleinsäureanhydrid bestehen, und das eine Grenzviskositätszahl $[\eta]$ von 0,05 bis 1,0 [dl/g] besitzt, und
(B) einem Copolymerisat, dessen Struktureinheiten aus statistisch verteilten polymerisierten Resten von Acrylamid, Acrylsäure und Maleinsäure bestehen, — wobei die Maleinsäurereste zumindest teilweise in Salzform vorliegen —, das eine Grenzviskositätszahl $[\eta]$ von 0,05—1,5 [dl/g] besitzt, und 65 bis 90 Mol-%, bezogen auf Polymer, Reste des Acrylamids und der Acrylsäure zusammen, und 10—35 Mol-% Reste der Maleinsäure enthält,
bestehen, wobei das Gewichtsverhältnis (A) : (B) = 1 : 2 bis 20 : 1 beträgt.

2. Verfahren zur Herstellung von Mikrokapseln gemäß Anspruch 1, dadurch gekennzeichnet, daß man

a) Gelatine im Gemisch mit Carboxymethylcellulose und anionischen Polymeren, die aus
   (A) einem Copolymerisat, dessen Struktureinheiten zu 65—90 Mol-%, bezogen auf Polymer, aus statistisch verteilten Resten des Acrylamids und 10—35 Mol-% Resten von Maleinsäure oder Maleinsäureanhydrid bestehen, und das eine Grenzviskositätszahl $[\eta]$ von 0,05 bis 1,0 [dl/g] besitzt, und
   (B) einem Copolymerisat, dessen Struktureinheiten aus statistisch verteilten polymerisierten Resten von Acrylamid, Acrylsäure und Maleinsäure bestehen, — wobei die Maleinsäurereste zumindest teilweise in Salzform vorliegen —, das eine Grenzviskositätszahl $[\eta]$ von 0,05—1,5 [dl/g] besitzt, und 65 bis 90 Mol-%, bezogen auf Polymer, Reste des Acrylamids und der Acrylsäure zusammen, und 10—35 Mol-% Reste der Maleinsäure enthält,
   bestehen, wobei das Gewichtsverhältnis (A) : (B) = 1 : 2 bis 20 : 1 beträgt,
   in Gegenwart des Kernmaterials in wäßrigem Medium durch Verdünnung, Abkühlung und/oder durch Änderung des pH-Wertes des Gemisches koazerviert,
b) die Hüllen der dabei entstehenden Mikrokapseln nach in saurem Medium bei pH-Werten zwischen 3,5 und 5,0 vorzunehmender Vorhärtung mit synthetischen Gerbstoffen danach mit Glutardialdehyd im schwach sauren oder basischen Medium bei pH-Werten zwischen 6,0 und 10,0 härtet und
c) schließlich die so erhaltene Mikrokapselsuspension entweder direkt trocknet oder die Mikrokapseln von der flüssigen Phase trennt und dann trocknet.

3. Verwendung von Mikrokapseln gemäß Anspruch 1 in Waschmitteln oder Badezusätzen.

**Claims**

1. Microcapsules which release the enclosed core material in the presence of water within a defined, narrow temperature range, characterised in that the wall material of the microcapsules consists of gelatin which is in admixture with carboxymethylcellulose and anionic polymers and has been hardened successively with synthetic tanning agents and with glutarodialdehyde, wherein the anionic polymers consist of

(A) a copolymer, of which the structural units consist, to an extent of 65—90 mole %, based on the polymer, of randomly distributed radicals of acrylamide and 10—35 mole % of radicals of maleic acid or maleic anhydride, and which has an intrinsic viscosity $[\eta]$ of 0.05 to 1.0 [dl/g], and

(B) a copolymer, of which the structural units consist of randomly distributed polymerised radicals of acrylamide, acrylic acid and maleic acid, — the maleic acid radicals being at least partly present in salt form — and which has an intrinsic viscosity $[\eta]$ of 0.05—1.5 [dl/g] and contains 65 to 90 mole %, based on the polymer, of radicals of acrylamide and acrylic acid, calculated together, and 10—35 mole % of radicals of maleic acid,

the weight ratio of (A) : (B) being 1 : 2 to 20 : 1.

2. Process for the production of microcapsules according to Claim 1, characterised in that

a) gelatin in admixture with carboxymethylcellulose and anionic polymers which consist of

(A) a copolymer, of which the structural units consist, to an extent of 65—90 mole %, based on the polymer, of randomly distributed radicals of acrylamide and 10—35 mole % of radicals of maleic acid or maleic anhydride, and which has an intrinsic viscosity $[\eta]$ of 0.05 to 1.0 [dl/g], and

(B) a copolymer, of which the structural units consist of randomly distributed polymerised radicals of acrylamide, acrylic acid and maleic acid — the maleic acid radicals being at least partly present in salt form — and which has an intrinsic viscosity $[\eta]$ of 0.05—1.5 [dl/g], and contains 65 to 90 mole %, based on the polymer, of radicals of acrylamide and acrylic acid, calculated together, and 10—35 mole % of radicals of maleic acid,

the weight ratio of (A) : (B) being 1 : 2 to 20 : 1,

is coacervated in the presence of the core material in an aqueous medium by diluting, cooling and/or by changing the pH value of the mixture,

b) the shells of the microcapsules thereby formed, after being pre-hardened with synthetic tanning agents, which pre-hardening is to be carried out in an acid medium at pH values of between 3.5 and 5.0, are then hardened with glutarodialdehyde in a weakly acid or basic medium at pH values of between 6.0 and 10.0 and

c) finally, the microcapsule suspension thus obtained is either dried directly or the microcapsules are separated from the liquid phase and then dried.

3. Use of microcapsules according to Claim 1 in washing agents or bath additives.

**Revendications**

1. Microcapsules qui libèrent la matière interne qu'elles renferment en présence d'eau dans un intervalle de température étroit et défini, caractérisées en ce que la matière constituant la paroi des microcapsules est formée de gélatine durcie successivement avec des tannins synthétiques ainsi qu'avec du dialdéhyde glutarique, en mélange avec de la carboxyméthylcellulose et des polymères anioniques, les polymères anioniques étant constitués par

(A) un copolymère dont les motifs structuraux sont formés à 65—90 moles %, par rapport au polymère, de restes de l'acrylamide à répartition statistique et à 10—35 moles %, de restes d'acide maléique ou d'anhydride ds'acide maléique, et qui possède un indice de viscosité limite $[\eta]$ de 0,05 à 1,0 [dl/g], et par

(B) un copolymère dont les motifs structuraux sont formés de restes polymérisés à répartition statistique d'acrylamide, d'acide acrylique et d'acide maléique, les restes d'acide maléique étant présents au moins partiellement sous la forme de sel —, qui possède un indice de viscosité limite $[\eta]$ de 0,05 à 1,5 [dl/g], et contient 65 à 90 moles %, par rapport au polymère, de restes d'acrylamide et d'acide acrylique considérés ensemble, et 10 à 35 moles % de restes d'acide maléique,

le rapport en poids (A) : (B) ayant une valeur de 1 : 2 à 20 : 1.

2. Procédé de production de microcapsules suivant la revendication 1, caractérisé en ce que

a) on effectue la coacervation de gélatine en mélange avec de la carboxyméthylcellulose et des polymères anioniques qui sont constitués par

    (A) un copolymère dont les motifs structuraux sont formés à 65—90 moles %, par rapport au polymère, de restes d'acrylamide à répartition statistique et à 10—35 moles % de restes d'acide maléique ou d'anhydride d'acide maléique, et qui possède un indice de viscosité limite $[\eta]$ de 0,05 à 1,0 [dl/g], et par

    (B) un copolymère dont les motifs structuraux sont constitués par des restes polymérisés à répartition statistique d'acrylamide, d'acide acrylique et d'acide maléique — les restes d'acide maléique étant présents au moins partiellement sous forme de sel —, qui posède un indice de viscosité limite $[\eta]$ de 0,05 à 1,5 [dl/g], et qui contient 65 à 90 moles %, par rapport au polymère, de restes d'acrylamide et d'acide acrylique considérés ensemble, et 10 à 35 moles % de restes d'acide maléique, le rapport en poids (A) : (B) ayant une valeur de 1 : 2 à 20 : 1,

en présence de la matière interne en milieu aqueux par dilution, refroidissement et/ou modification de la valeur de pH du mélange,

b) on fait durcir les enveloppes des microcapsules ainsi produites après durcissement préalable devant être effectué en milieu acide à des valeurs de pH comprises entre 3,5 et 5,0 avec des tannis synthétiques, puis avec du dialdéhyde glutarique en milieu faiblement acide ou basique à des valeurs de pH comprises entre 6,0 et 10,0 et

c) finalement, on sèche directement la suspension obtenue de microcapsules ou bien on sépare les microcapsules de la phase liquide et on les sèche ensuite.

3. Utilisation de microcapsules suivant la revendication 1 dans des détergents ou des additifs pour le bain.